# EUROPEAN PATENT SPECIFICATION

(11) **EP 0 598 719 B1**
(45) Date of publication and mention of the grant of the patent: **16.09.1998**
(21) Application number: 91915386.6
(22) Date of filing: 31.07.1991
(51) Int. Cl.: A61K 47/48, A61K 9/127

(54) **RECEPTOR CONJUGATES FOR TARGETING DRUGS AND OTHER AGENTS**
REZEPTOR-KONJUGATE ZUM TARGETING VON ARZNEIWIRKSTOFFEN UND ANDEREN WIRKSUBSTANZEN
CONJUGUES COMPRENANT UN RECEPTEUR, DESTINES A CIBLER DES MEDICAMENTS ET AUTRES AGENTS

(43) Date of publication of application: 01.06.1994
(73) Proprietor: Antex Biologics, Inc., Gaithersburg, MD 20879 (US)
(72) Inventor: KRIVAN, Howard, C., Plymoth Montserrat (MS); BLOMBERG, Arne, Lennart, Ingemar Villa Vallmo, S-223 60 Lund (SE)
(74) Representative: Brown, John David
(86) International application number: US9105422
(87) International publication number: WO9302709

(56) References cited:
- EP-A- 0 036 277
- EP-A- 0 409 527
- WO-A-86/05789
- WO-A-88/03810
- WO-A-89/01519
- WO-A-91/05771
- DE-A- 3 711 724
- PROCEEDINGS OF THE NATIONAL ACADEMY OF SCIENCES,USA vol. 85, 1988, WASHINGTON D.C.,USA pages 6157 - 6161; KRIVAN ET AL: 'MANY PULMONARY PATHOGENIC BACTERIA BIND SPECIFICALLY TO THE CARBOHYDRATE SEQUENCE GALNACB1-4GAL FOUND IN SOME GLYCOLIPIDS' cited in the application
- CHEMICAL ABSTRACTS, vol. 104, 1986, Columbus, Ohio, US; abstract no. 56285E, TROUET ET AL: 'DRUG TARGETING:A LONG WAY FROM CONCEPT TO CLINICAL APPLICATIONS' page 355 ;column 1 ;
- CHEMICAL ABSTRACTS, vol. 101, 1984, Columbus, Ohio, US; abstract no. 43522T, TROUET ET AL: 'CELL TARGETING OF PRIMAQUINE' page 318 ;column 2 ;
- CHEMICAL ABSTRACTS, vol. 99, 1983, Columbus, Ohio, US; abstract no. 48473C, NICOLAU ET AL: 'LIPOSOMES AS CARRIERS FOR GENE TRANSFER IN VIVO' page 114 ;column 2 ;
- CHEMICAL ABSTRACTS, vol. 102, 1985, Columbus, Ohio, US; abstract no. 183015A, DASGUPTA ET AL: 'RECEPTOR-MEDIATED UPTAKE OF ASIALOGANGLIOSIDE LIPOSOMES:SUBCELLULAR DISTRIBUTION OF THE LIPOSOMAL MARKER IN ISOLATED LIVER CELL TYPES' page 401 ;column 2 ;

## Description

### Technical Field

The present invention relates generally to conjugates comprising an agent, such as an anti-infective, coupled to a receptor which binds a microorganism, and to methods for making and using these conjugates.

### Background of the Invention

A reoccurring problem in medicine is that, due to the lack of specificity of the agents used for treatment of illnesses, the patient is often the recipient of a new set of maladies from the therapy. This scenario is common and has occurred in the treatment of infections due to pathogenic microorganisms.

The conventional approach to attempting to minimize adverse side-effects of an anti-microbial agent, such as a drug, to a patient has been to prepare a myriad of chemical derivatives in which moieties are added and/or deleted. The derivatives are then assessed for their effectiveness as well as their toxicity. Such an approach to minimizing adverse side-effects has been costly, time-consuming, and not always successful.

Due to the difficulties in the current approaches to the preparation of anti-microbial agents which exhibit minimal side effects, there is a need in the art for such agents. The present invention fills this need, and further provides other related advantages.

### Summary of the Invention

Briefly stated, the present invention provides a variety of conjugates useful as *in vitro* inhibitors, such conjugates are also disclosed as therapeutic agents, e.g., for the treatment of infections due to pathogenic microorganisms. The microorganism receptor-agent conjugates comprise at least one agent coupled to at least one glycolipid, the glycolipid being capable of selectively binding a bacterial microorganism.

In one embodiment, the conjugate includes an agent which is an anti-infective. Preferred anti-infectives include antibiotics, synthetic drugs and steroids. In another embodiment, the conjugate includes an agent which is a molecule that induces neutralization of the microorganism, for example, by stimulating the production of antibodies.

Within a related aspect, the present invention provides methods for inhibiting a microorganism in an in vitro biological preparation. In one embodiment, the method comprises contacting an in vitro biological preparation with an effective amount of a microorganism receptor-agent conjugate, wherein the conjugate comprises at least one agent coupled to at least one glycolipid, the glycolipid being capable of selectively binding a bacterial microorganism.

There are disclosed methods for the treatment of infections due to pathogenic microorganisms. In one embodiment, the method comprises administering to a warm-blooded animal an effective amount of a conjugate described above, wherein the glycolipid is capable of selectively binding a pathogenic microorganism. A preferred warm-blooded animal is a human.

These and other aspects will become evident upon reference to the following detailed description and attached drawings.

### Brief Description of the Drawings

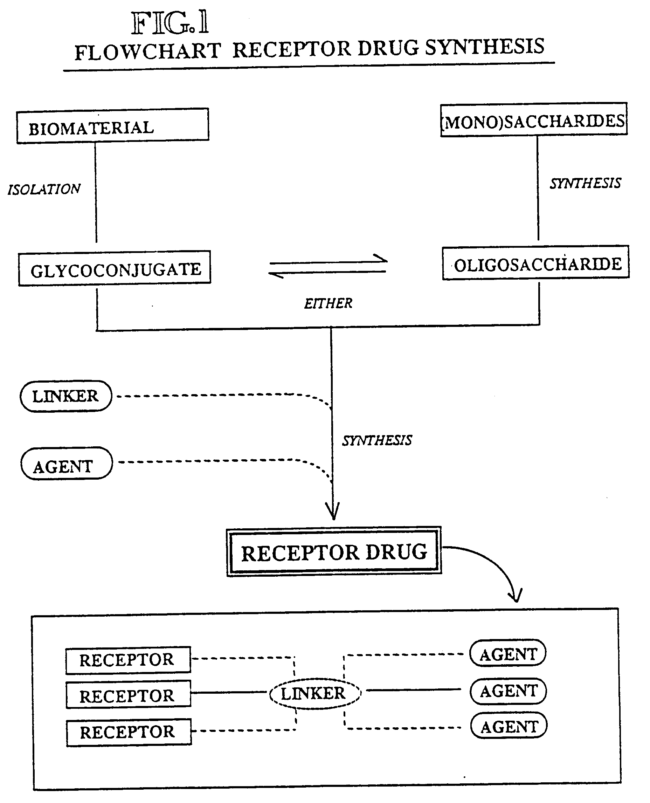

Figure 1 depicts a flowchart illustrating a procedure for the preparation of microorganism conjugates of the present invention in which the agent portion is a drug.

Figure 2 shows a diagram illustrating the cross-linking of amoxicillin to the glycolipid receptor asialo-GM₁ using a photoreactive linker.

### Detailed Description of the Invention

As noted above, the present invention provides microorganism receptor-agent conjugates and methods for using the conjugates *in vitro*. There is also disclosed use of the conjugates as therapeutic agents, e.g., for the treatment of infections due to pathogenic microorganisms. These conjugates, in which at least one agent is coupled to at least one glycolipid, have enormous potential as potent anti-microbial compositions. This is due to the selectivity imparted to the conjugate by the glycolipid portion. The selectivity of the glycolipid permits increased targeting and specificity for the pathogen. In addition, targeting of anti-microbial agents by using the conjugates of the present invention minimizes the dosage and adverse side-effects, such as the accumulation of toxic drugs in vital organs, in a patient.

Glycolipids on a host cell may function as receptors for the recognition and attachment of microorganisms to the host cell. The active part of a glycolipid receptor, i.e., the minimum binding epitope, appears generally to he the carbohydrate moiety. Therefore, the targeting portion ("glycolipid receptor") of the conjugates of the present invention comprises a glycolipid. Glycolipid receptors of the present invention include purified receptors or portions thereof, synthetically prepared receptors or portions thereof, and derivatives of receptors or portions thereof. Such a glycolipid receptor is capable of selectively binding a bacterial microorganism. Recognition and attachment of microorganisms to host cells is the result of specific interactions, between molecules on the microorganisms and microorganism receptors on the host cells, that permit the receptors to selectively bind microorganisms. More than one pathogenic microorganism may bind to the same epitope, e.g., carbohydrate sequence, in order to infect cells. Conversely, a microorganism may have unique receptor specificities. In either situation, a microorganism is infecting a host cell by selectively binding to a microorganism receptor. The binding of many microorganisms to glycolipid receptors is generally half maximal (B_{1/2 max}) within a range of about 0.02-0.2 micrograms of purified receptor. For example, for *Streptococcus pneumoniae* about 0.05 µg of immobilized asialo-GM₁ results in half maximum binding, and *Helicobacter pylori* requires about 0.1 µg of immobilized receptor.

Glycolipid receptors for microorganisms may be purified from host cells by standard biochemical techniques. For example, glycolipids may be purified by the methods described by Karlsson (Meth. Enzymol. 138:212-219, 1987). Briefly, body fluid or cells are extracted with one or more organic solvents and the extract is subjected to mild alkaline degradation. Following neutralization and dialysis, the lipids and glycolipids are separated by a series of chromatography techniques, e.g., silicic acid and ion-exchange chromatography. The preparative steps are typically checked by thin-layer chromatography. (TLC). Purified, intact receptors may be used to prepare the conjugates of the present invention. Alternatively, it will be evident to one skilled in the art that, using chemical, and/or enzymatic, reagents and techniques, an intact receptor may be cleaved (to yield a portion thereof) and/or structurally modified (to yield a derivative of an intact receptor or portion thereof).

A representative example is the purification of asialogangliosides (Krivan et al., Proc. Natl. Acad. Sci. USA 85:6157-6161, 1988). Briefly, fucosylasialo-GM₁ and asialo-GM₁ were prepared from bovine brain gangliosides by hydrolysis in 25 mM H₂SO₄ for 1.5 hours at 80°C. The hydrolysis was neutralized with NH₄OH and dried under nitrogen, the residue was dissolved in chloroform/methanol/water (60:30:4.5, vol/vol), and non-glycosphingolipid contaminants were removed by Sephadex G-25 column chromatography (Wells et al., Biochemistry 2:1259-1263, 1963). Fucosylasialo-GM₁ and asialo-GM₁ were separated from residual gangliosides by column chromatography on DEAE Sepharose and further purified by continuous thin-layer chromatography (Young et al., Meth. Enzymol. 138:125-132, 1987) on preparative silica gel G plates with chloroform/methanol/water (75:18:2.5, vol/vol) as the mobile phase. Asialo-GM₂ was obtained after digestion of asialo-GM₁ with bovine testes β-galactosidase (0.5 unit/ml) for 36 hours at 37°C in 0.1 M acetate buffer (pH 5.0) containing 0.2% sodium taurocholate. Polar contaminants and detergent were removed by Sephadex G-25 and DEAE-Sepharose column chromatography, respectively.

Alternatively, once the receptor structure has been identified, it may be prepared synthetically using chemical, and/or enzymatic, reagents and techniques. Similarly, the carbohydrate moiety of a receptor may be isolated from host cells or, following structural determination, prepared synthetically. Similar to the discussion above regarding purified receptors or portions thereof, structurally modified receptors or portions thereof may be prepared synthetically.

Briefly, in the case of enzymatic synthesis of carbohydrates, natural unprotected mono-, di- or oligosaccharides and sialic acids are used as starting materials. Properly activated derivatives thereof in the anomeric center may have to be used. The glycoside synthesis is hereafter carried out with the help of specific enzymes. In the case of chemical synthesis, the same starting materials described above or derivatives thereof can be used. In this case, proper preactivation of the anomeric center together with proper specific protection of the remaining hydroxyls has to be performed prior to use in specific glycoside synthesis. For example, five hydroxyl groups of a hexose may be converted to -OR₁ through -OR₅, with R₂-R₅ representing protective groups. If the compound is used as the glycosyl donor, R₁ is a group that is suitable for activation in a glycoside synthesis by a different catalyst. Examples of such groups are halides, sulfur derivatives, acetimidates and orthoesters. Conversely, if the compound is used as the glycosyl acceptor, R₁ can be a protecting group as described below. R₁ can also be chosen in a way such that it can be converted in a later step into a group as described above. A third possibility is that R₁ is a ligand suitable for further coupling to other compounds.

Protecting groups from diverse arts may be employed when the derivative is used as the glycosyl donor. Commonly used protecting groups are, for example, acetyls, benzyls, benzoates and acetals. If the compound is used as the glycosyl acceptor, one or several of the hydroxyls is unprotected in order to render them accessible in the glycoside synthesis. It is well known in the art to choose the protecting groups such that the hydroxyls are selectively deblocked in order to continue an oligosaccharide synthesis. R₂-R₅ may also be other protected carbohydrate residues or other substituents or functional groups. The glycosyl donor and the glycosyl acceptor may be reacted together in the presence of a suitable catalyst to create the desired glycosidic bond. Depending on how the protecting groups, the anomeric group, the catalyst and the reaction condition are chosen, stereoselectivity and the desired stereochemistry can be obtained. If desired, this protected product can be deprotected to the free oligosaccharide. If further reactions are to be carried out, this can be done by proper selection of the starting materials which facilitates other glycosidation reaction by selectively manipulating the protecting groups and the anomeric center. This can be done both with a stepwise or a blockwise approach. The hydroxyl substituents may also be changed into other functional groups or attached to a ligand suitable for coupling.

Other representative examples of receptors for microorganisms include the following molecules. *Cryptococcus neoformans*, *Candida albicans*, and other fungi bind specifically to the glycosphingolipid lactosylceramide (Jimenez-Lucho et al., Infect. and Immun. 58:2085-2090, 1990). Isolated as well as synthetic lactosylceramides are commercially available (e.g., Sigma Chem. Co., St. Louis, Mo. and Calbiochem-Behring, La Jolla, Calif.). *Mycoplasma pneumoniae* bind specifically to sulfatide and other sulfated glycolipids (Krivan et al., J. Biol. Chem. 264:9283-9288, 1989), as well as to sialylated glycoproteins (Roberts et al., J. Biol. Chem. 264:9289-9293, 1989). Sulfated glycolipids may be purified as described by Krivan et al. or obtained commercially (e.g., Supelco). Similarly, sialylated glycoproteins may be purified according to Roberts et al. or obtained commercially (e.g., Sigma Chem. Co.). Influenza virus binds specifically to sialic acid (Weis et al., Nature 333:426-431, 1988). Rotaviruses bind specifically to the neutral glycosphingolipid asialo-GM₁ (Willoughby et al., J. Virol. 64:4830-4835, 1990).

In addition to at least one glycolipid, the conjugates of the present invention include at least one agent which directly or indirectly inhibits microorganisms. A variety of agents are suitable. For example, in one embodiment, one or more agents which are cytotoxic to a microorganism are coupled to a microorganism receptor to create a conjugate that may be termed a "receptor drug." Preferred agents are the classes of anti-infectives, such as antibiotics and synthetic drugs, that are efficacious in the treatment of infections due to pathogenic microorganisms. Representative antibacterial agents include aminoglycosides, polymyxins, sulfonamides, metronidizole, trimethoprim-sulfamethoxazole, and penicillins. A representative antiviral agent is acyclovir. Representative antifungal agents include amphotericin B, nystatin and 5-fluorocytosine. Representative antiparasitic agents include pentamidine and nitoimidazoles. Other agents which may be useful include steroids such as corticosteroids, e.g., prednisone, prednisilone and dexamethasone. The receptor drugs of the present invention provide an efficient drug targeting system that specifically eliminates pathogens by, for example, "fooling" the pathogen into binding to an artificial receptor (i.e., one coupled to a cytotoxic agent) rather than to a natural receptor on a host cell. Alternatively, pathogens may already be attached to host cells and the receptor drugs of the present invention may bind to the pathogens via, for example, extra, specific molecules on the pathogens that are not bound to the receptors.

The conjugates of the present invention, in another representative embodiment, include a molecule that induces neutralization of a microorganism. For example, the agent may be a molecule which stimulates the production of antibodies. Because, for example, carbohydrate receptors for pathogenic microorganisms are typically small and occur naturally on host cells, they are usually not immunogenic. However, a microorganism receptor can be coupled to a carrier molecule, such as keyhole limpet hemocyanin, that confers immunogenicity. Consequently, when a pathogenic microorganism binds to this type of receptor conjugate, the pathogen becomes attached via the receptor portion of the conjugate to a molecule which stimulates the production of antibodies by the host. Binding of antibodies to the pathogen via the conjugate sets in motion a sequence of events, the end result of which is neutralization of the pathogen.

An agent may be coupled to, e.g., covalently bonded to, a glycolipid receptor either directly or indirectly, e.g., via a linker group. A direct reaction between an agent and a receptor is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group, e.g., a halide, on the other.

Alternatively, it may be desirable to couple an agent and a receptor via a linker group. A linker group can function as a spacer to distance a receptor from an agent in order to avoid interference with binding capabilities, e.g., by steric hindrance or conformational changes. A linker group can also serve to increase the chemical reactivity of a substituent on an agent or a receptor, and thus increase the coupling efficiency. An increase in chemical reactivity may also facilitate the use of agents, or functional groups on agents, which otherwise would not be possible. A carboxyl group, for example, may be activated. Activation of a carboxyl group includes formation of an "active ester," such as a succinimidyl ester. The term "active ester" is known to refer to esters which are highly reactive in nucleophilic substitution reactions.

It will be evident to one skilled in the art that a variety of bifunctional or polyfunctional reagents, both homo- and hetero-functional (such as those described in the Pierce Chemical Co. catalog), may be employed as the linker group. Coupling may be effected, for example, through amino groups, carboxyl groups, sulfhydryl groups or oxidized carbohydrate residues. There are numerous references describing such methodology, e.g., U.S. Patent No. 4,671,958, to Rodwell et al.

Where an agent is more potent when free from the receptor portion of the conjugates of the present invention, it may be desirable to use a linker group which is cleavable, e.g., bio-cleavable. A number of different cleavable linker groups have been described previously. The mechanisms for release of an agent from these linker groups include cleavage by reduction of a disulfide bond (e.g., U.S. Patent No. 4,489,710, to Spitler), by irradiation of a photolabile bond (e.g., U.S. Patent No. 4,625,014, to Senter et al.), by hydrolysis of derivatized amino acid side chains (e.g., U.S. Patent No. 4,638,045, to Kohn et al.), by serum complement-mediated hydrolysis (e.g., U.S. Patent No. 4,671,958, to Rodwell et al.), and acid-catalyzed hydrolysis (e.g., U.S. Patent No. 4,569,789, to Blattler et al.).

It may be desirable to couple more than one agent to a glycolipid receptor. In one embodiment, multiple molecules of an agent are coupled to one receptor molecule. In another embodiment, more than one type of agent may be coupled to one receptor. Regardless of the particular embodiment, conjugates with more than one agent may be prepared in a variety of ways. For example, receptors with multiple sites for attachment of agents can be coupled directly, or linkers which provide multiple sites for attachment can be used. Alternatively, a carrier can be used. A carrier may bear the agents in a variety of ways, including covalent bonding either directly or via a linker group. Suitable carriers include proteins such as albumins (e.g., U.S. Patent No. 4,507,234, to Kato et al.), peptides and polysaccharides such as aminodextran (e.g., U.S. Patent No. 4,699,784, to Shih et al.). A carrier may also bear an agent by noncovalent bonding or by encapsulation, such as within a liposome (e.g., U.S. Patent Nos. 4,429,008 and 4,873,088). Similarly, it may be desirable to include more than one receptor in a conjugate. The above discussion regarding agent is applicable here as well. For example, a conjugate may be produced in which multiple molecules of a receptor are coupled to a liposome carrier (e.g., incorporated into a liposome membrane) which encapsulates (i.e., traps inside the liposome vesicle) an agent or agents. A representative example is asialo-GM₁-containing liposomes encapsulating amoxicillin, metronidazole, and/or bismuth subsalicylate.

As noted above, there are disclosed methods of using the conjugates described above. In one aspect, the method comprises administering to a warm-blooded animal, such as a human, an effective amount of these conjugates. It will be evident to one skilled in the art that the site of infection will be the most important factor in determining not only the choice of the particular conjugate, but also the route by which it should be administered. For instance, a fungal infection of the skin may be treated by topical administration and a bacterial infection of the ears by oral administration. Methods of administration include oral, intravenous, intramuscular, topical and rectal. For oral administration, the conjugates may be in pill, capsule or liquid form. For any method of administration, the conjugates may be combined with a physiologically acceptable carrier or diluent, such as water or physiological saline.

By administering to a warm-blooded animal an effective amount of a conjugate of the present invention, treatment of an infection due to a pathogenic microorganism is effected. The causative agent of an infection may be bacteria, viruses, mycoplasma, fungi or parasites. Representative bacteria include the gram-negative, gram-positive, anaerobes, spirochetes, mycobacteria and actinomyces. Representative viruses include RNA and DNA viruses, e.g., herpes, cytomegalovirus, influenza, hepatitis, RSV and HIV. Representative mycoplasmataceae include *M. pneumonies, M. hominus, Ureaplasma and Acoleplasma*. Representative fungi include *Candida, Cryptococcus, Coccidioides, Sporothrix, Aspergillus* and *Histoplasma*. Representative parasites include protozoa (e.g., trhichomonas, pneumocystis and entomoeba) and helminths (e.g., nematodes and trematodes). Other pathogenic microorganisms include the chlamydia/rickettsia group, e.g., *C. trachomatis, C. psittaci, C. pneumoniae* (TWAR), *Rickettsia* and *Coxiella* bacteria.

The mechanism by which a pathogenic microorganism is neutralized depends upon the type of agent in a particular conjugate. For example, certain agents, such as those which stimulate the production of antibodies, work in conjunction with existing host cell defense mechanisms. Alternatively, other agents, such as those which are cytotoxic, may inactivate the microorganism more directly.

The precise dose for a particular conjugate may vary, depending upon the agent and the receptor used. In particular, agents vary with respect to their potency and receptors vary with respect to binding affinity. Generally, however, an effective amount of a conjugate of the present invention will be from about 0.1 to about 10 mg per kg body weight. It will be evident to one skilled in the art how to determine the optimal effective dose for a particular conjugate. For example, the effective amount may be determined based upon *in vitro* experiments, followed by *in vivo* studies. Such methodologies include measuring the minimal inhibitory concentration (MIC) and minimal bacteriocidal concentration (MBC). The principle of the MIC, for example, is to determine the lowest or minimal concentration of antimicrobial agent that is required to inhibit the growth of a particular microorganism *in vitro*, and is usually expressed in micrograms per milliliter. Approved standards have been published by the National Committee for Clinical Laboratory Standards (National Committee for Clinical Laboratory Standards. Methods for Dilution Antimicrobial Susceptibility Tests for Bacterial That Grow Aerobically. Tentative Standard NCCLS Publication M7-T2. Villanova, PA:NCCLS, 1988). For determination of the optimal effective amount of receptor drug *in vivo*, dilution of serum that is inhibitory or bacteriocidal to an organism isolated from a patient receiving the drug is analyzed. Proposed guidelines for performing a serum bacteriocidal test have been published by the National Committee for Clinical Laboratory Standards (National Committee for Clinical Laboratory Standards. Methodology on Serum Bacteriocidal Test. Proposed Guideline. NCCLS Document M21-P. Villanova, PA:NCCLS, 1987).

The particular conjugate administered is dependent on the nature of the infection or microorganism that is to be targeted. Determining the nature of an infection may be accomplished by a variety of known techniques, such as assays using body fluids. For example, several immunologic methods for detection of microbial antigens are available, including enzyme immunoassay, latex agglutination, coagglutination, counterimmunoelectrophoresis, fluoroimmunoassay, and radioimmunoassay. All of these methods detect a particular microbial antigen, i.e., a toxin, that infers the presence of a particular microorganism and cause of the disease. Other methods which may be used alone or in conjunction with immunologic assays include direct culture, microscopy, biochemical and antimicrobial susceptibility testing. In the embodiment where the agent is an antibiotic or synthetic drug, a compound which is efficacious for a particular infection is coupled to the appropriate receptor. For example, amoxicillin is used to treat pneumonia because it affects the growth of *Streptococcus pneumoniae*. In preparing a receptor drug for treating pneumonia, amoxicillin may be covalently coupled to asialo-GM₁ oligosaccharide, the carbohydrate structure which is the receptor for the organism. Other examples of receptor drugs within the present invention include a lactosylceramide-amphotericin B conjugate for use against fungi (e.g., *Candida* or *Cryptococcus*) and a sulfatide-tetracycline or sulfatide-erythromycin conjugate for use against mycoplasmas. Examples of the drugs currently of choice for representative microorganisms are listed in Table 1 below.

**Table 1**

| Drug of Choice | Microorganism | Class of Organism |
|---|---|---|
| Amphotericin B | *Candida*, *Cryptococcus* | Fungi (yeast) |
| Metronidazole | Trichomonas | Protozoan |
| Amoxicillin | *Helicobacter pylori* (formerly *Campylobacter pylori*) | Bacteria |
| Ampicillin/Amoxicillin | *Streptococcus pneumoniae* | Bacteria |
| Tetracycline/Erythromycin | *Mycoplasma pneumoniae* | Mycoplasma |
| Tetracycline | *Chlamydia trachomatis* | Bacteria |
| Acyclovir/Ganciclovir | Herpes/Cytomegalovirus | Virus |

As noted above, conjugates of the present invention may also be used for *in vitro* inhibition of a microorganism, such as in a biological preparation. The term "biological preparation" includes biological samples taken *in viv*o and *in vitro* (either with or without subsequent manipulation), as well as those prepared synthetically. Representative examples of biological preparations include cells, tissues, solutions and bodily fluids, such as (or from) blood, urine, saliva, sweat, synovial, cerebrospinal and tears. Briefly, one or more of the conjugates are added to a biological preparation. The precise optimal concentration may vary, depending upon the particular conjugate used. Generally, however, a concentration of about 0.1 to 100 mg per ml will be effective. One of the uses of this aspect of the present invention is to prevent microbial colonization of a biological preparation during its storage.

The following examples are offered by way of illustration and not by way of limitation.

### EXAMPLES

### Example 1

### Preparation of Asialo-GM₂-Amoxicillin

### A. Preparation of Asialo-GM₂-Oligosaccharide

Melting points are corrected. Reactions were performed under nitrogen. Concentrations were performed at <40°C (bath). Optical rotations were recorded at 25°C with a Perkin-Elmer 241 polarimeter. Thin-layer chromatography was performed on silica gel 60 F₂₅₄ (Merck, Darmstadt, FRG) using the following eluant systems: A, 4:3:3:2 ethyl acetate: acetic acid: methanol: water, B, 10:5:1 chloroform: methanol: water, C, 11:9:2 chloroform: methanol: water. The spots were visualized by charring with 5% aqueous sulfuric acid. Silica gel chromatography was performed on Matrex silica Si, 60A, 20-45 MY (Amicon Corporation, Danvers, Ma. 01923, U.S.A.), using solvent system D, 5:1 methylene chloride: pyridine and E, 10:5:3:1:1 chloroform: methanol: dioxane, water: pyridine, Sulfuryl chloride/triflic acid reagent was made 1 M in toluene containing 10% diethylether. Organic solvents were of p.a. quality and distilled over appropriate drying agents.

### p-Methylphenyl 3,4,6-tri-O-p-chlorobenzyl-2-deoxy-2-phthalimido-1-thio-β-D-galactopyranoside (1):

Into a stirred solution of p-methylphenyl-2-azido-3,4,6-tri-O-chlorobenzyl-2-deoxy-1-thio-β-D-galactopyranoside (5.00 g) in 1:1 pyridine/triethylamine (200 ml) at room temperature was bubbled H₂S until saturation. The flask was sealed and stirring was continued for 2 hours. Then nitrogen was flushed through the solution, and phtalic anhydride (3.0 g) in methylene chloride (100 ml) was added. The mixture was stirred overnight. Then acetic anhydride (50 ml) in toluene (100 ml) was added. After 2 hours, water (50 ml) was added. The organic phase was washed with water, saturated sodium bicarbonate and 1 M sulfuric acid and evaporated. The resulting syrup was chromatographed in 7/1 toluene/ethyl acetate. Crystallization of appropriate fractions from diethylether/isooctane gave pure (1) (3.89 G, 67.5%), mp 63-70°C, [α]_{D} + 70.1° (*c* 1.0 chloroform).

### Ethyl 4-O-β-galactopyranosyl-1-thio-β-D-glucopyranoside (2):

To a mixture of β-lactose peracetate (50 g), ethanethiol (6.9 g, 822 ml) and 200 ml dry CH₂Cl₂ was added BF₃/Et₂O (8.5 g, 7.3 ml) at RT. After 2 hours, TLC (toluene/ethyl acetate 2/3) showed no more reaction. The mixture was shaken with ca 500 ml 1M NaOH. The organic layer was directly evaporated and taken up in methanol (150 ml), then NaOMe in methanol (10 ml, 0.5M) was added and the mixture was stirred overnight at RT. The TLC (ethyl acetate/acetic acid/methanol/water 12/3/3/3) yields an Rf 0.41. The reaction mixture was neutralized with Dowex (50w x 8, H⁺), filtered and concentrated. The residue was recrystallized from ethanol (300 ml). Yield 16.4 g, 56%, mp 191-192°C.

### Ethyl 4-O-(4,6-O-benzylidene-β-D-galactopyranosyl)-1-thio-β-D-glucopyranoside (3):

A mixture of (2) (3.00 g) and bensaldehyde (30 ml) was stirred for 1 hour at room temperature. Then formic acid (30 ml) was added, and stirring was maintained for a further 25 minutes. The clear solution was poured into diethylether (400 ml) during stirring. After 1 hour, the solid was filtered off and dissolved in methanol (50 ml) during heating. After cooling diethylether (25 ml) was added. Crystals were obtained after standing overnight (3.09 g, 84%), 240-242°C, [α]_{D} - 49.3°.

### Ethyl 4-O-(4,6-O-benzylidene-2,3-di-O-p-chlorobenzyl-β-D-galactopyranosyl-2,3,6-tri-O-p-chlorobenzyl-1-thio-β-D-glucopyranoside (4):

Treatment of (3) (2.00 g) with p-chlorobenzyl chloride (3.0 ml) and sodium hydride (1.4 g) in DMF (50 ml) at 0°C under nitrogen gave a single spot on TLC (toluene/ethyl acetate 4/1, Rf 0.39). Partitioning between toluene and 1 M sulfuric acid and water, and crystallization from dichloromethane/ethyl acetate/isooctane gave 3.57 g (4), 77%, mp 179-183°C, [α]_{D} + 10.9° (*c* 1.0, chloroform).

### Ethyl 4-O-(6-O-benzyl-2,3-di-O-p-chlorobenzyl-β-D-galactopyranosyl)-2,3,6-tri-O-p-chlorobenzyl-1-thio-β-D-glucopyranoside (5):

Compound (4) (100 mg) in THF (10 ml) containing molecular sieves 3Å) 600 mg) at room temperature under nitrogen was treated with NaCNBH₃ (100 mg) and HCl (saturated in diethyl ether) as described. After 2 hours, TLC (toluene/ethyl acetate 4/1, Rf 0.55) showed complete reaction. The mixture was filtered, partitioned between dichloromethane and sodium bicarbonate and water. Pure (5) was obtained after crystallization from ethyl acetate/isooctane (82 mg, 82%), mp 137-139°C, [α]_{D} + 25.6°.

### 2-(p-Nitrophenyl)ethyl 4-O-(6-O-benzyl-2,3-di-O-p-chlorobenzyl-β-D-galactopyranosyl)-2,3,6-tri-O-p-chlorobenzyl-β-D-glucopyranoside (6):

A solution of (5) (500 mg) in methylene chloride (20 ml) was treated with bromine (50 µ) and molecular sieves 4Å (5.0 b) at 0°C during stirring. After 30 minutes, TLC (toluene/ethyl acetate 4/1) indicated that no starting material remained, and excess bromine was destroyed with two drops of cyclohexene. The slurry was added dropwise to a stirred mixture of 2-(4-nitrophenyl)ethanol (300 mg) and freshly activated zinc chloride (5.0 g) in methylene chloride (10 ml), while maintaining nitrogen atmosphere and 0°C. After two hours, the mixture was diluted with methylene chloride, filtered, washed with water and 1 M sulfuric acid, dried and concentrated. The resulting syrup was chromatographed in isooctane/ethyl acetate 1/1. Fractions containing pure material of Rf 0.53 was pooled and concentrated (340 mg, 62%). Nmr analysis showed this to be the desired (6). Crystals of (6) were obtained from diethyl ether/isooctane, mp 110-112°C, [α]_{D} + 22.8°.

### 2-(p-Nitrophenyl)ethyl 4-O-(3,4,6-tri-O-p-chlorobenzyl-2-deoxy-2-phthalimide-β-D-galactopyranosyl) 1-O-(6-O-benzyl-2,3-di-O-p-chlorobenzyl-β-D-galactopyranosyl)-2,3,6-tri-O-p-chlorobenzyl-β-D-glucopyranoside (7):

To an ice cooled solution of disaccharide (6) (71 mg, 1 eq) and thioglycoside (1) (58 mg, 1.2 eq) in dry methylene chloride (5.0 ml) containing molecular sieves 4Å (100 mg) was added SO₂Cl₂/HOTf reagent (0.30 ml, 5 eq) under nitrogen during stirring. The mixture was stirred for 2 hours during which the temperature was allowed to rise to 10°C. Then pyridine (100 µl) was added and the mixture was stirred for another hour at room temperature. The mixture was filtered, partitioned between ethyl acetate and aqueous sodium bicarbonate, dried (MgSO₄) and concentrated. After silica gel chromatography in toluene/ethyl acetate 59% of (7) was obtained.

### 2-(p-Nitrophenyl)ethyl 4-O-(3,4,6-tri-O-p-chlorobenzyl-2-acetamido-2-deoxy-β-D-galactopyranosyl)-4-O-(6-O-benyl-2,3-di-O-p-chlorobenzyl-β-D-galactopyranosyl)-2,3,6-tri-O-p-chlorobenzyl-β-D-glucopyranoside (8):

To a stirred solution of trisaccharide (7) (400 mg) in toluene/95% ethanol, 1/10 (10 ml) was added hydrazine hydrate (0.3 ml) and acetic acid (0.2 ml). The mixture was refluxed overnight, cooled, concentrated and co-evaporated with toluene/ethanol. The residue was treated with acetic anhydride/pyridine 1/1 (5 ml) for 30 minutes at room temperature. Concentration, partitioning between toluene and water, drying (MgSO₄) and concentration gave a syrup. The syrup was chromatographed on silica gel in n-heptane/ethyl acetate 1/1 (Rf 0.35). Appropriate fractions were pooled and concentrated to give (8) in 52% yield.

### 2-(p-Trifluoroacetamidophenyl)ethyl 4-O-(2-acetamido-2-deoxy-β-D-galactopyranosyl)-4-O-β-D-galactopyranosyl-β-D-glucopyranoside (9):

To compound (8) (50 mg) in THF (2 ml), acetic acid (1 ml) and water (0.1 ml) was added zinc dust (100 mg) and the mixture was stirred at 0°C under nitrogen. Then a solution of CuSO₄ x 5 H₂O (100 mg.ml, 0.2 ml) was added. After 30 minutes TLC (n-heptane: ethyl acetate 1:1, Rf 0.28) showed complete reaction. The mixture was filtered, diluted with CH₂Cl₂, washed with aqueous sodium bicarbonate, water, dried (MgSO₄) and concentrated. The residue was dissolved in CH₂Cl₂ (3 ml). The solution was cooled to 20°C and pyridine (40 µl) and trifluoroacetic anhydride (20 µl) were added. After 10 minutes TLC (n-heptane: ethyl acetate 1:1, Rf 0.13) showed complete reaction. The mixture was concentrated to dryness and dissolved in ethyl acetate: ethanol: acetic acid: water 4:2:1:1 containing sodium acetate (50 mg) and hydrogenolyzed over Pd/C (10%, 50 mg) at atmospheric pressure for 8 hours as described. Complete debenzylation was indicated by TLC (ethyl acetate/methanol/acetic acid/water, 12/3/3/2, Rf 0.15). Purification by C-18 chromatography as described before gave 80% of (9). The structures of compounds (1) to (9) are shown below.

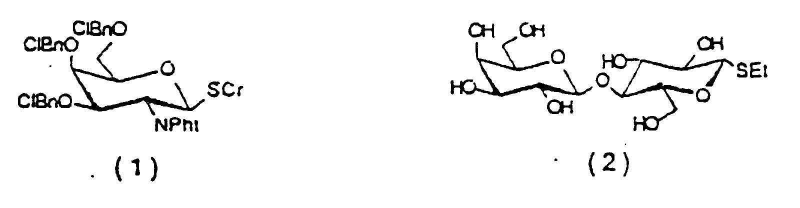

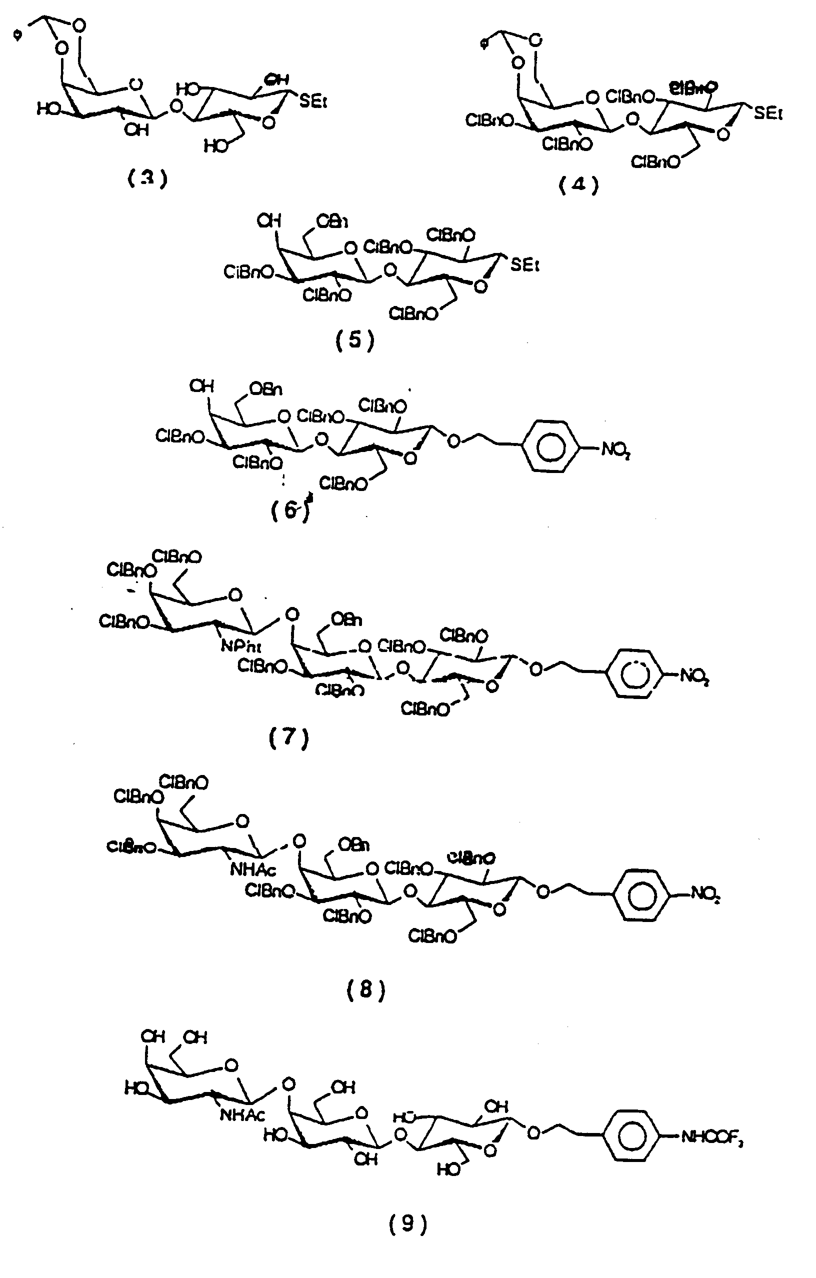

### B. Preparation of Asialo-GM₂ Oligosaccharide-Amoxicillin

### 2-(p-Aminophenyl)ethyl 4-O-(2-acetamido-2-deoxy-β-D-galactopyranosyl)-4-O-β-D-galactopyranosyl-β-D-glucopyranoside (10):

100 mg of compound (9) is dissolved in 10 ml 25% ammonia at 50°C. The mixture was left for an hour and is then put directly on a C 18 column and washed with water until the pH reaches about 9. The column was then eluted with 30% methanol. Ninhydrin positive fractions were pooled and partly evaporated to remove the bulk of methanol. The remaining solution was subjected to freeze-drying which gave a white fluffy powder pure by TLC. The yield is 95%.

### 2-(p-iso-Thiocyanatophenyl)ethyl 4-O-(2-acetamido-2-deoxy-β-D-galactopyranosyl)-4-O-β-D-galactopyranosyl-β-D-glucopyranoside (11):

100 mg of compound (10) is dissolved in 10 ml 70% ethanol at room temperature. To the solution is added a twofold excess of thiophosgen and the solution is stirred for five minutes. After that enough ion exchanger is added to rise the pH to about 5 (Dowex 1 x 2 OH form). The ion exchange is then filtered off and washed with water. The filtrate is evaporated to remove most of the ethanol. The remaining solution is then freeze-dried to dryness which leaves a white powder essentially pure by TLC and NMR. The yield is about 50%.

### 2-(p-Amoxicillin thiourea phenyl) ethyl 4-O-(2-acetamido-2-deoxy-β-D-galactopyranosyl)-4-O-β-D-galactopyranosyl-β-D-glucopyranoside (12):

50 mg of compound (11) is dissolved in 5 ml DMF. An equimolar amount of Amoxicillin is added and the solution is left for two days at room temperature which gives a clear yellow solution. On TLC (EtOAc:MeOH:H₂O:HOAc 12:3:3:2) only traces of the reactants can be seen and one major product together with small amounts of byproducts. The solvent is evaporated with the help of a vacuum pump slightly above room temperature. The remaining solid is dissolved in water and chromatographed on a C 18 column. It is first eluted with water and thereafter with 30% methanol. The desired fractions are pooled and freeze-dried after evaporation of the methanol. The structure of the substance is confirmed by NMR and FAB/MS. The yield is 62%. The structures of compounds (9) to (12) are shown below.

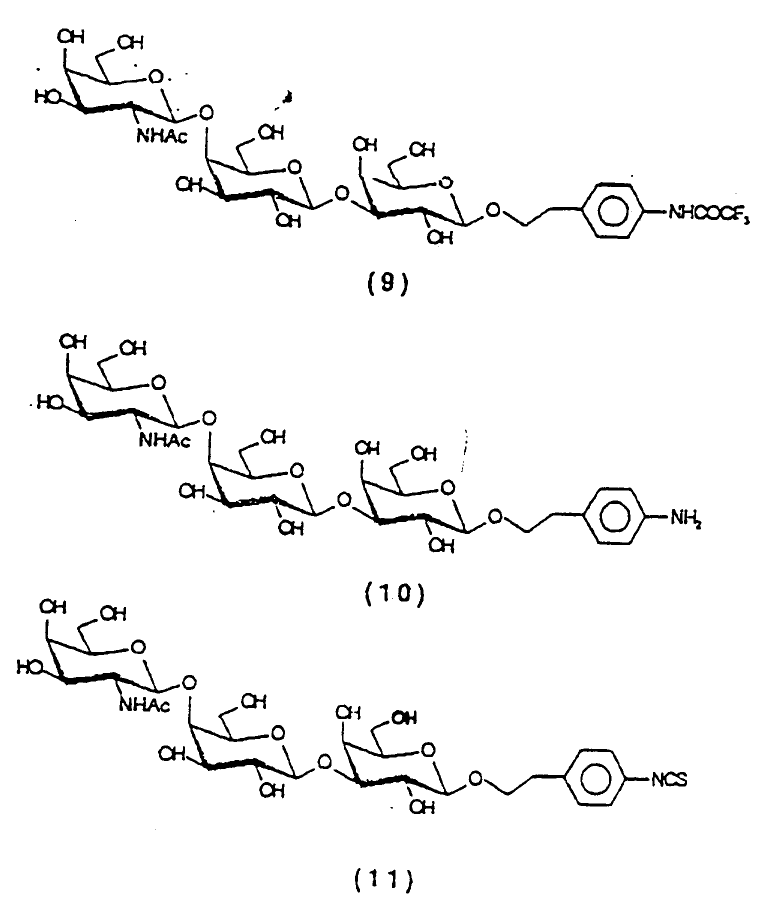

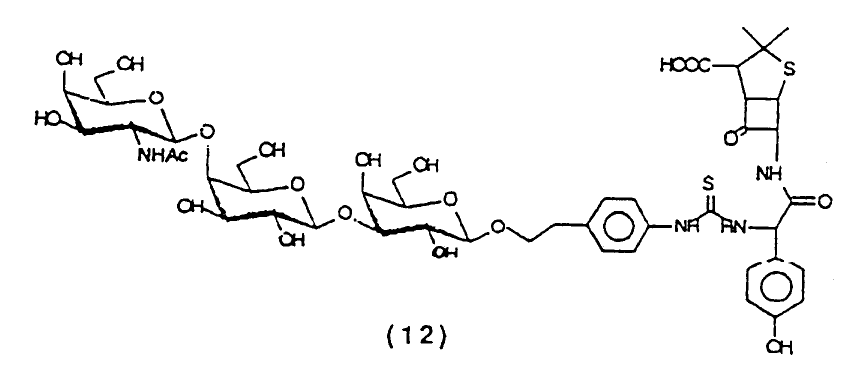

### Example 2

### Preparation of Asialo-GM₁-Amoxicillin

### A. Asialo-GM₁

Asialo-GM₁ can be purified from gangliosides as described above or purchased (BioCarb Chemicals, Lund, Sweden).

### B. Preparation of Asialo-GM₁-Amoxicillin Using A Hetero-Bifunctional Reagent

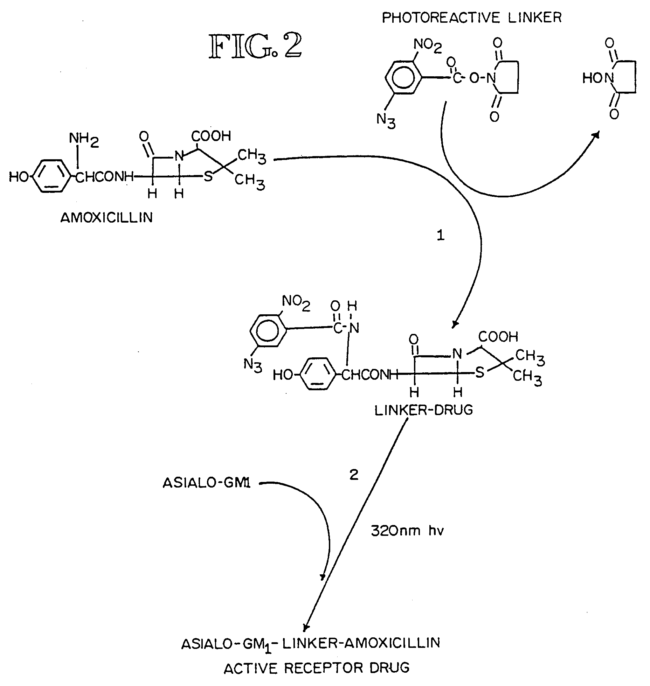

Asialo-GM₁ (BioCarb Chemicals, cat. # 65/03) and other relevant glycolipids are made to a concentration of 10 mg/ml in HPLC-grade dimethylsulfoxide (DMSO) and stored at 4°C until use. The photo-heterobifunctional reagent ANB-NOS (Pierce, cat. # 21551) is dissolved in the dark in HPLC-grade DMSO at a concentration of 300 mg/ml and stored in the dark at 4°C until use. Amoxicillin (Sigma, cat. # A-8523, lot # 29F0730) is dissolved in DMSO to a concentration of 120 mg/ml. In the dark, ANB-NOS-DMSO and Amoxicillin-DMSO are mixed in a 1:1 ratio (wt/wt) and incubated at room temperature for one hour (step 1 in Figure 2). After this incubation in the dark, asialo-GM₁ is added to the reaction mixture in a ratio of 12:1 ANB-NOS-Amoxicillin to asialo-GM₁. The reaction mixture is exposed to a sunlamp (G.E. bulb # RSM-6) for 15 minutes where the reaction mixture will change from yellow to amber resulting in Amoxicillin-Asialo-GM₁ (step 2 in Figure 2). The reaction is approximately 30%-50% efficient.

### Example 3

### Preparation of Liposomes Containing Microorganism Receptor and Encapsulating Antimicrobial Agents

Receptor liposomes were prepared based on the methodologies described in Gruner et al. (Biochemistry 24:2833-2842,1985) and Dahlgren et al. (J. Immunol. Meth. 44:223-234, 1981). In brief, liposomes were prepared according to the procedure of Gruner et al. using a ratio of cholesterol/phosphatidylcholine/sphingolipid (1:1:1, molar ratio) as described by Dahlgren et al. When glycolipids and glycolipid receptors were used, sphingolipid was reduced by the corresponding amount of glycolipids used. Drug(s) were added in excess prior to liposome formation.

Lipids and glycolipids (90 mg cholesterol, Matreya; 180 mg phosphatidylcholine, Sigma; 90 mg sphingomyelin, Sigma; 45 mg asialo-GM₁, BioCarb Chemicals; 45 phosphatidylethanolamine, Sigma) were dissolved in 100 ml of chloroform in a round-bottom flask and rotoevaporated. The resulting film was dissolved in 50 ml of diethylether. Aqueous drug(s) were added in Hepes buffer (e.g., bismuth subsalicylate, Crescent Chem. Co., at 25 mg/ml in buffer containing 72.5 mM NaCl, 72.5 mM KCl and 10 mM Hepes, pH 7.4). The remaining 250 ml of diethylether was added. While sonicating in a water bath maintained at room temperature or lower, the ether was removed by a stream of N₂. Ether removal was discontinued when about 99% of the ether is gone and the volume remaining is equal or less than the volume of the aqueous phase added. Liposome formation was complete at this stage as evidenced by the appearance of a white waxy "cake" in the buffer.

The liposome material and buffer were transferred to glass tubes and centrifuged at room temperature for 20 min. at 12,000 RPM (SA-600 rotor). The liposome pellet was washed three times with the Hepes buffer (50-100 ml per wash). After the final wash, the liposomes were resuspended in Hepes buffer (e.g., so that each dose will be 100 µl per dose). Alternatively, the liposomes may be resuspended in a concentrated amount of bismuth (25 mg/ml) for a final concentration of 20 mg/ml.

### Example 4

### Inhibition of Streptococcus pneumoniae by Asialo-GM₁-Amoxicillin In Vitro

Determination of the minimum inhibitory concentration (MIC) was done according to recommendations published by the National Committee for Clinical Laboratory Standards (Tentative Standard NCCLS Publication M7-T2, Villanova, Pa., NCCLS, 1988). Both amoxicillin and amoxicillin-asialo-GM₁ (prepared according to Example 2) were tested for bacteriostatic and bacteriocidal levels using a clinical isolate of *Streptococcus pneumoniae*. Stock solutions of amoxicillin and amoxicillin-asialo-GM₁ were diluted to 10 µg/ml in Trypticase soy broth without glucose (T-soy from Difco). Serial two-fold dilutions were made from stocks in a series of 16 tubes each containing 1 ml of medium such that tube 1 contained 5 µg/ml through tube 16 which contained 0.0001 µg/ml of antibiotic. To each of these tubes was added 0.05 ml of a suspension of *S. pneumoniae* (approximately 1.5 x 10⁸) organisms/ml using a 0.5 McFarland standard. T-soy broth with no organisms and with organisms and no antibiotics served as negative and positive controls, respectively. All tubes were incubated at 37°C in 5% CO₂/95% air for 18 hours and read for turbidity and MIC. For determination of bacteriocidal levels (MBC), 0.001 ml was taken from each tube showing no visible growth, inoculated onto 5% sheep blood agar plates and incubated an additional 18 hours. A 99% reduction in colony count compared to control tubes was considered bacteriocidal (MBC). The results of the comparison of amoxicillin and amoxicillin-asialo-GM₁ are shown in Table 2.

**Table 2**

| Comparison of amoxicillin and amoxicillin-asialo-GM₁ against *Streptococcus pneumoniae* as measured by minimum inhibitory concentration (MIC) and minimum bacteriocidal concentration (MBC). | | |
|---|---|---|
| Drug | MIC (lg/ml) | MBC (lg/ml) |
| Amoxicillin | 0.04 | 0.04 |
| Amoxicillin-Asialo-GM₁* | 0.005 | 0.005 |

| | | |
|---|---|---|
| *Prepared using an ANB-NOS-Amoxicillin to glycolipid ratio of 1:1 | | |

### Example 5

### Inhibition of Campylobacter (Helicobacter) pylori In Vivo

Clinical studies suggest that *Helicobacter Pylori*-Like Organisms (HPLO) may cause duodenal ulcers, gastritis and hypochlorhydria. Moreover, HPLO may be responsible for unexplained vomiting in man. Several studies in Rhesus monkeys have demonstrated the presence of organisms closely resembling HPLO found in humans. In the monkeys used in this experiment, small curved rod-shaped bacteria measuring 3-4 µm long and 0.5-10.0 µm wide were seen in close proximity to the mucosal epithelial cells in 8/29 monkeys. These bacteria were very similar to *C. pylori* observed in humans and were therefore called HPLO. The effect of these bacteria on radiation induced vomiting and gastric suppression is unknown.

It is interesting that, although gastritis is known to be associated with gastric ulcer, duodenal ulcers and gastric cancer, its treatment remains symptomatic, and little if any improvements is observed after administration of typical antacids and/or histamine H₂ antagonists. In contrast, administration of bismuth salts and of several antibiotics has improved gastritis, while eradicating HPLOs. However, the prolonged use of large doses of antibiotics may lead to eradication of the normal flora and to the development of bacterial resistance and recurrence of infection is extremely frequent.

### A. Treatment with Asialo-GM₁-Amoxicillin

Two domestic born male rhesus monkeys, *Macaca Mulatta*, in which HPLO is present in gastric biopsies and weighing 3-7 kg, were housed in individual stainless steel cages in conventional holding rooms of an AALAC accredited animal facility. The two infected monkeys were treated by administering blindly and t.i.d. either placebo or 7 mg/kg of asialo-GM₁-amoxicillin (prepared according to Example 2) diluted in Tang, a drink that is avidly consumed by monkeys and allows reliable oral administration of medications. The animals were treated for two days only, but HPLO were cultured only from gastric biopsies obtained immediately after the end of the treatment in the animal receiving placebo and not in the one treated with the receptor conjugate.

### B. Treatment With Liposomes Containing Microorganism Receptor and Encapsulating Antimicrobial Agents

Liposomes containing a specific glycolipid receptor for *Helicobacter pylori* (HP) and encapsulating amoxicillin, metronidazole and bismuth subsalicylate (7, 7, and 10 mg/kg, respectively) were prepared according to Example 3. *In vitro*, these liposomes inhibited the growth of HP in broth by a factor of 4 to 10 as compared with free antimicrobial agents alone. Gastroduodenoscopies and mucosal biopsies were performed under anesthesia in six colony-bred rhesus monkeys spontaneously infected with HP-Like Organisms (HPLO). The animals were then treated orally for five days with the liposome complex and the endoscopies were repeated 4, 17 and 60 days later. Mucosal biopsies were grown on HP medium, tested for urease activity, or fixed for light microscopy, and the presence or absence of infection was assessed blindly. At each time, plasma IgG levels (as a percentage of the mean plus 3 standard deviations of 40 non-infected children) were determined against a pool of *H. pylori* antigens in an assay with >95% sensitivity and specificity for human infection. Within one week after the end of treatment, infection in the corpus, but not in the antrum, was decreased or suppressed, and plasma IgG did not change significantly (0.85 ± 0.14 vs. 0.69 ± 0.09; NS).

From the foregoing, it will be evident that, although specific embodiments of the invention have been described herein for purposes of illustration, various modifications may be made without deviating from the and scope of the invention.

## Claims

1. A microorganism receptor-agent conjugate, comprising at least one agent coupled to at least one glycolipid, said glycolipid being capable of selectively binding a bacterial microorganism.

2. The microorganism receptor-agent conjugate of Claim 1, wherein said glycolipid is asialo-GM₁.

3. The microorganism receptor-agent conjugate of Claim 16, wherein said glycolipid is asialo-GM₂.

4. The microorganism receptor-agent conjugate of Claim 1, 2 or 3, wherein said agent is an antibiotic.

5. The microorganism receptor-agent conjugate of Claim 4, wherein said antibiotic is a penicillin.

6. The microorganism receptor-agent conjugate of Claim 4, wherein said penicillin is amoxicillin.

7. The microorganism receptor-agent conjugate of any one of the preceding claims, wherein said conjugate comprises a carrier coupled to at least one glycolipid capable of selectively binding a bacterial microorganism, and said carrier bears at least one agent.

8. The microorganism receptor-agent conjugate of Claim 7, wherein said agent is an antibiotic.

9. A microorganism receptor-agent conjugate of any one of Claims 1 to 8 for use as a therapeutic agent.

10. A microorganism receptor-agent conjugate of any one of Claims 1 to 8 for use for the treatment of an infection due to a pathogenic bacterial microorganism.

11. Use of the microorganism receptor-agent conjugate of any one of Claims 1 to 8 for the manufacture of a medicament for the treatment of a bacterial infection.

12. Use according to Claim 11, in which said agent is an antibiotic.

13. Use according to Claim 12, in which said antibiotic is amoxicillin and said bacterial infection is by *Streptococcus pneumoniae*.

14. Use according to Claim 12, in which said antibiotic is amoxicillin and said bacterial infection is by *Campylobacter pylori* or *Helicobacter pylori*.

15. A method for inhibiting a bacterial microorganism in an *in vitro* biological preparation, comprising contacting said *in vitro* biological preparation with an effective amount of the microorganism receptor-agent conjugate of any one of Claims 1 to 8.

16. The method according to Claim 15, in which said agent is an antibiotic.

17. The method according to Claim 16, in which said antibiotic is amoxicillin and said bacterial infection is by *Streptococcus pneumoniae*.

18. The method according to Claim 16, in which said antibiotic is amoxicillin and said bacterial infection is by *Campylobacter pylori* or *Helicobacter pylori*.

## Patentansprüche

1. Ein Mikroorganismusrezeptor-Agens-Konjugat, das wenigstens ein Agens umfaßt, das an wenigstens ein Glykolipid gekoppelt ist, wobei besagtes Glykolipid in der Lage ist, selektiv einen bakteriellen Mikroorganismus zu binden.

2. Das Mikroorganismusrezeptor-Agens-Konjugat nach Anspruch 1, wobei besagtes Glykolipid asialo-GM₁ ist.

3. Das Mikroorganismusrezeptor-Agens-Konjugat nach Anspruch 1, wobei besagtes Glykolipid asialo-GM₂ ist.

4. Das Mikroorganismusrezeptor-Agens-Konjugat nach Anspruch 1, 2 oder 3, wobei besagtes Agens ein Antibiotikum ist.

5. Das Mikroorganismusrezeptor-Agens-Konjugat nach Anspruch 4, wobei besagtes Antibiotikum ein Penicillin ist.

6. Das Mikroorganismusrezeptor-Agens-Konjugat nach Anspruch 4, wobei besagtes Penicillin Amoxicillin ist.

7. Das Mikroorganismusrezeptor-Agens-Konjugat nach einem der vorangehenden Ansprüche, wobei besagtes Konjugat einen Carrier umfaßt, der an wenigstens ein Glykolipid gekoppelt ist, das in der Lage ist, selektiv einen bakteriellen Mikroorganismus zu binden, und besagter Carrier wenigstens ein Agens trägt.

8. Das Mikroorganismusrezeptor-Agens-Konjugat nach Anspruch 7, wobei besagtes Agens ein Antibiotikum ist.

9. Ein Mikroorganismusrezeptor-Agens-Konjugat nach einem der Ansprüche 1 bis 8 zur Verwendung als ein therapeutisches Mittel.

10. Ein Mikroorganismusrezeptor-Agens-Konjugat nach einem der Ansprüche 1 bis 8 zur Verwendung zur Behandlung einer Infektion aufgrund eines pathogenen bakteriellen Mikroorganismus.

11. Verwendung eines Mikroorganismusrezeptor-Agens-Konjugats nach einem der Ansprüche 1 bis 8 zur Herstellung eines Arzneimittels zur Behandlung einer bakteriellen Infektion.

12. Verwendung nach Anspruch 11, bei der besagtes Agens ein Antibiotikum ist.

13. Verwendung nach Anspruch 12, bei der besagtes Antibiotikum Amoxicillin ist und besagte bakterielle Infektion durch Streptococcus pneumoniae hervorgerufen ist.

14. Verwendung nach Anspruch 12, bei der besagtes Antibiotikum Amoxicillin ist und besagte bakterielle Infektion durch Campylobacter pylori oder Helicobacter pylori hervorgerufen ist.

15. Ein Verfahren zur Inhibition eines bakteriellen Mikroorganismus in einem biologischen in-vitro Präparat, welches umfaßt, daß besagtes biologisches in-vitro-Präparat mit einer wirksamen Menge des Mikroorganismusrezeptor-Agens-Konjugats nach einem der Ansprüche 1 bis 8 in Kontakt gebracht wird.

16. Das Verfahren nach Anspruch 15, wobei besagtes Agens ein Antibiotikum ist.

17. Das Verfahren nach Anspruch 16, wobei besagtes Antibiotikum Amoxicillin ist und besagtes bakterielle Infektion durch Streptococcus pneumoniae hervorgerufen ist.

18. Verfahren nach Anspruch 16, wobei besagtes Antibiotikum Amoxicillin ist und besagte bakterielle Infektion durch Campylobacter pylori oder Helicobacter pylori hervorgerufen ist.

## Revendications

1. Conjugué récepteur de micro-organisme-agent comprenant au moins un agent couplé à au moins un glycolipide, ledit glycolipide étant capable de lier sélectivement un micro-organisme bactérien.

2. Conjugué récepteur de micro-organisme-agent selon la revendication 1, dans lequel ledit glycolipide est asialo-GM₁.

3. Conjugué récepteur de micro-organisme-agent selon la revendication 1 , dans lequel ledit glycolipide est asialo-GM₂.

4. Conjugué récepteur de micro-organisme-agent selon la revendication 1, 2 ou 3, dans lequel ledit agent est un antibiotique.

5. Conjugué récepteur de micro-organisme-agent selon la revendication 4, dans lequel ledit antibiotique est une pénicilline.

6. Conjugué récepteur de micro-organisme-agent selon la revendication 4, dans lequel ladite pénicilline est l'amoxicilline.

7. Conjugué récepteur de micro-organisme-agent selon l'une quelconque des revendications précédentes, dans lequel ledit conjugué comprend un porteur couplé à au moins un glycolipide capable de lier sélectivement un micro-organisme bactérien, et ledit porteur porte au moins un agent.

8. Conjugué récepteur de micro-organisme-agent selon la revendication 7, dans lequel ledit agent est un antibiotique.

9. Conjugué récepteur de micro-organisme-agent selon l'une quelconque des revendications 1 à 8 pour utilisation en tant qu'agent thérapeutique.

10. Conjugué récepteur de micro-organisme-agent selon l'une quelconque des revendications 1 à 8 pour utilisation pour le traitement d'une infection due à un micro-organisme pathogène bactérien.

11. Utilisation du conjugué récepteur de micro-organisme-agent selon l'une quelconque des revendications 1 à 8 pour la fabrication d'un médicament pour le traitement des infections bactériennes.

12. Utilisation selon la revendication 11, dans laquelle ledit agent est un antibiotique.

13. Utilisation selon la revendication 12, dans laquelle ledit antibiotique est l'amoxicilline et ladite infection bactérienne est par *Streptococcus pneumoniae*.

14. Utilisation selon la revendication 12, dans laquelle ledit antibiotique est par l'amoxicilline et ladite infection bactérienne est par *Campylobacter pylori* ou *Helicobacter pylori*.

15. Méthode pour inhiber un micro-organisme bactérien dans une préparation biologique *in vitro,* comprenant la mise en contact de ladite préparation biologique *in vitro* avec une quantité efficace du conjugué récepteur de micro-organisme-agent selon l'une quelconque des revendications 1 à 8.

16. Méthode selon la revendication 15, dans laquelle ledit agent est un antibiotique.

17. Méthode selon la revendication 16, dans laquelle ledit antiblotique est l'amoxicilline et ladite infection bactérienne est par *Streptococcus pneumoniae*.

18. Méthode selon la revendication 16, dans laquelle ledit antibiotique est l'amoxicilline et ladite infection bactérienne est par *campylobacter pylori* ou *Helicobacter pylori*.
